# EUROPEAN PATENT APPLICATION

(11) **EP 2 508 161 A1**
(43) Date of publication of application: **10.10.2012**
(21) Application number: 10834353.4
(22) Date of filing: 10.11.2010
(51) Int. Cl.: A61J 3/00

(54) **MEDICINE ASSORTMENT SYSTEM AND CART**

(30) Priority: 03.12.2009 JP 2009275114
(71) Applicant: Panasonic Corporation, Kadoma-shi Osaka 571-8501 (JP)
(72) Inventor: GOTOU, Makoto, Osaka 540-6207 (JP); MATSUKAWA, Yoshihiko, Osaka 540-6207 (JP); TANIMOTO, Takanobu, Osaka 540-6207 (JP); NAKAMURA, Tohru, Osaka 540-6207 (JP)
(74) Representative: Grünecker, Kinkeldey, Stockmair & Schwanhäusser
(86) International application number: PCT/JP2010/006599
(87) International publication number: WO 2011/067897

(57) **Abstract**

A medicine collecting system (11) includes a cart (14) having a plurality of housing units (23) that each house a tray (12), and a dispenser (13) that dispenses a first medicine to the tray housed in each housing unit of the cart (14) according to prescription information that indicates the type of a medicine to be dispensed to each tray and the number of the medicines and is set for each tray. A memory unit (28) of the system (11) stores, for each tray, the prescription information with housing unit identification information that identifies the housing unit housing the tray to which the first medicine is dispensed by the dispenser (13), in association with one another. The system (11) includes a medicine rack processing unit (33) that identifies the prescription information containing information on a second medicine to be supplied to each tray after dispensing of the first medicine and determines the housing unit identification information corresponding to the identified prescription information, and an LED (26) that presents information that identifies the housing unit (23) indicated by the housing unit identification information.

## Description

### [Technical Field]

The present invention relates to a medicine collecting system that assists in collecting of medicines to a tray and a cart used in to the medicine collecting system.

### [Background Art]

In order to collects medicines reliably and easily, a variety of medicine collecting apparatuses have been proposed. A medicine set-up system 1 shown in FIG. 12 is a system that can easily collect and check medicines (see, for example, PTL 1).

The medicine set-up system 1 includes a tray 2 sectioned into a plurality of regions 3 and a display unit 4 that displays item information on medicines. The item information on the medicines, which is displayed on the display unit 4, is information on medicines to be collected in the regions 3 of the tray 2.

A medicine manager collects the medicines to the regions 3 of the tray 2 according to the item information displayed on the display unit 4. The regions 3 each have a medicine reading unit 5, so that the medicine set-up system 1 can check whether or not there is an error in collecting of the medicines collected to the tray 2.

The medicine manager can move the medicines collected to the fixed tray 2 to other carrier trays and carry the carrier trays to a ward or a hospital room.

According to another medicine collecting method, a dispenser dispenses general medicines that can be dealt with by persons who have neither nursing qualification nor a pharmacist's license to individual trays according to prescription information automatically and mechanically.

### [Citation List]

### [Patent Literature]

[PTL 1]
   Japanese Unexamined Patent Application Publication No.2004-70803

### [Summary of Invention]

### [Technical Problem]

The conventional medicine set-up system 1 uses the fixed tray and thus, medicines need to be shifted, resulting in that the operation disadvantageously becomes complicated. For example, in the case of collecting medicines by use of the dispenser and the medicine set-up system 1 in combination, the dispenser directly dispenses medicines to a plurality of carrier trays, while the medicine set-up system 1 requires the medicine manager to collect medicines from the fixed tray to other carrier trays. For this reason, when attempting to supply the medicines collected by the medicine set-up system 1 to the individual carrier trays to which the medicines have been already dispensed by the dispenser, the medicine manager must search the carrier trays for patients requiring the supply. This tray searching operation becomes a heavy burden on the medicine manager. In addition, it is likely to supply the medicines to the carrier tray for another patient by mistake.

Thus, an object of the present invention is to provide a medicine collecting system that enables the medicine manager to easily supply a second medicine to a tray holding a dispensed first medicine.

### [Solution to Problem]

To attain the above-mentioned object, a medicine collecting system according to one aspect of the present invention is a medicine collecting system that assists in collecting of medicines, the system including a cart having a plurality of housing units each configured to house a tray to which a first medicine is dispensed according to prescription information, a memory unit configured to store, for each tray, cart information that identifies the cart, the prescription information and housing unit identification information that identifies the housing unit housing the tray to which the first medicine is dispensed, in association with one another, a determining unit configured to check, from information of the memory unit, for presence or absence of a second medicine to be supplied to each tray housed in each housing unit of the cart identified on the basis of the cart information, and determine, according to the prescription information, the housing unit identification information which identifies the housing unit needed to contain the second medicine; a housing presenting unit configured to indicate the housing unit identified by the housing unit identification information determined by the determining unit; and a medicine detecting unit configured to detect a code of a medicine, wherein the determining unit configured to check for presence or absence of the second medicine on the basis of the code detected by the medicine detecting unit.

A cart according to another aspect of the present invention is a cart used for a medicine collecting system that assists in collecting of a medicine, the cart comprising: a plurality of housing units each configured to house a tray to which a first medicine is dispensed; a plurality of light emitting units each provided at a position corresponding to each of the housing units; a cart processing unit configured to light the light emitting unit corresponding to the housing unit which contains the tray to be supplied a second medicine on the basis of externally-received information including information of the second medicine, and a medicine presenting unit presenting configured to present a type and a number of the second medicines to be supplied to the tray corresponding to the externally-received information.

### [Advantageous Effects of Invention]

According to the present invention, the medicine manager can easily and correctly supply the second medicine to the tray among the plurality of the trays housed in the cart, the tray holding the dispensed first medicines.

### [Brief Description of Drawings]

[FIG. 1] FIG. 1 is a functional block diagram showing a configuration of a medicine collecting system in accordance with Embodiment 1 of the present invention.
[FIG. 2] FIG. 2 is a perspective view of a carrier cart in accordance with Embodiment 1 of the present invention.
[FIG. 3] FIG. 3 is a view showing a dispenser and the cart at collecting of medicines in accordance with Embodiment 1 of the present invention.
[FIG. 4] FIG. 4 is a flow chart describing operations of the dispenser in accordance with Embodiment 1 of the present invention.
[FIG. 5] FIG. 5 is a view showing a collecting list stored in a managing unit in accordance with Embodiment 1 of the present invention.
[FIG. 6] FIG. 6 is a view showing a medicine rack and a cart at supply of a medicine in accordance with Embodiment 1 of the present invention.
[FIG. 7] FIG. 7 is a flow chart showing operations of the medicine rack in accordance with Embodiment 1 of the present invention.
[FIG. 8] FIG. 8 is a view showing a collecting list stored in the medicine rack in accordance with Embodiment 1 of the present invention.
[FIG. 9] FIG. 9 is a functional block diagram showing a configuration of a medicine collecting system in accordance with Embodiment 2 of the present invention.
[FIG. 10] FIG. 10 is a perspective view of a carrier cart in accordance with Embodiment 2 of the present invention.
[FIG. 11] FIG. 11 is a flow chart describing operations of the cart in accordance with Embodiment 2 of the present invention.

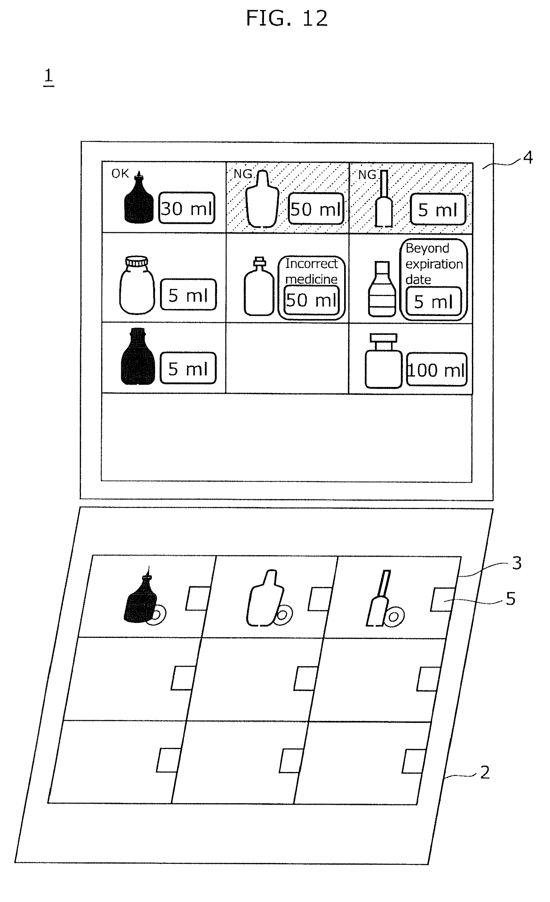

[FIG. 12] FIG. 12 is a view showing a conventional medicine set-up system.

### [Description of Embodiments]

The following describes embodiments for implementing the present invention with reference to the drawings. It is to be noted that, in the following description, the same numerals are assigned in the same structure and the explanation is omitted accordingly.

### (Embodiment 1)

A medicine collecting system according to Embodiment 1 of the present invention includes a carrier cart that houses trays dispensed by a dispenser. Here, each of the trays dispensed by the dispenser holds one or more first medicines which are also dispensed by the dispenser. The medicine collecting system transmits information, to the medicine manager, on one or more trays in a medicine rack of the carrier cart, each of which needs to be supplied one or more second medicines.

By use of the medicine collecting system according to Embodiment 1, the medicine manager can easily and correctly supply the second medicine to the tray that holds the medicine dispensed by the dispenser.

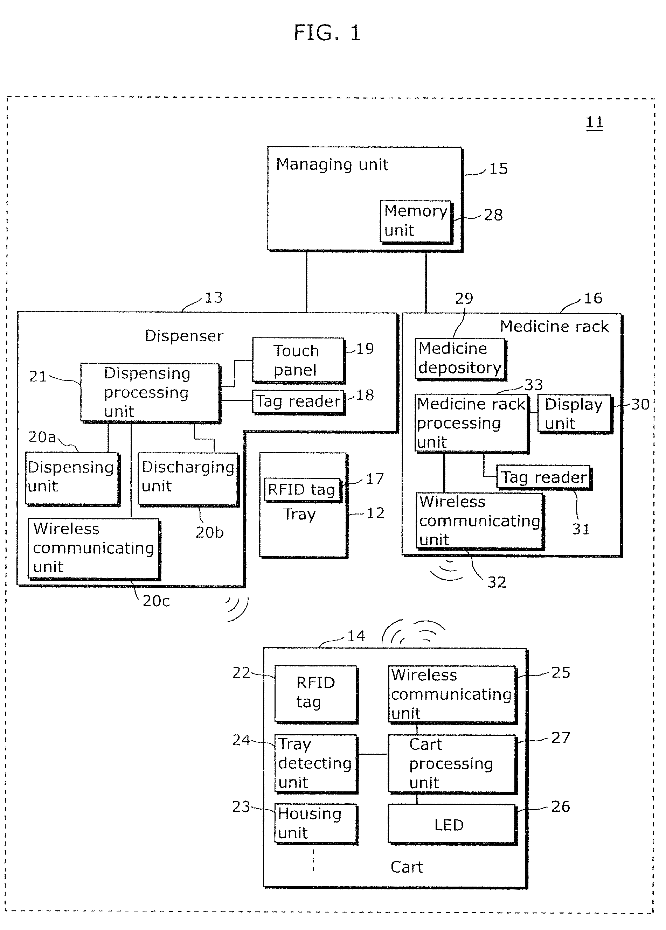

FIG. 1 is a functional block diagram showing a structure of a medicine collecting system 11 according to Embodiment 1.

The medicine collecting system 11 includes a cart 14, a dispenser 13, a medicine rack 16 and a managing unit 15.

The cart 14 has a plurality of housing units 23, each housing a tray 12.

The dispenser 13 dispenses the first medicine(s) to the tray 12 according to prescription information indicating a type and a number of each of the medicines to be dispensed to the tray 12. That is, the dispenser 13 automatically dispenses the first medicine(s) to the tray 12 according to the prescription information set for the tray 12. Then, the dispenser 13 houses step by step a plurality of the trays 12, each dispensed the first medicines, housing units 23 of the cart 14, respectively.

The medicine rack 16 holds the second medicines.

The managing unit 15 includes a memory unit 28 that stores housing unit position information (housing unit address). The housing unit position information (housing unit address) herein is information indicating the position of the housing units 23 housing the trays 12 to which the first medicine is dispensed by the dispenser 13. The memory unit 28 is an apparatus that stores a medicine collecting list for each tray. The memory unit 28 stores a cart number, the housing unit address, collecting information, a prescription number and prescription information, in association with one another, for each tray. The cart number is cart information for identifying the cart. The housing unit address is the housing unit position information for identifying the position of the housing unit, that is, housing unit identification information. The collecting information is information indicating the types and the numbers of the first medicines dispensed by the dispenser 13. The prescription number is prescription identification information for identifying prescription information. The prescription information is information indicating the type and the number of the medicines to be collected to each tray.

When a tag reader 31 of a medicine rack 16 detects the cart number (cart information for identifying the cart) stored in an RFID (Radio Frequency IDentification) tag 22 in the cart 14, a medicine rack processing unit 33 of the medicine rack 16 calculates the housing unit address of the housing unit 23 housing the tray 12 that needs to be supplied the second medicine. Through processing in the medicine rack processing unit 33, information on the tray 12 is shown to the medicine manager by means of a display unit 30 or the like.

The first medicines are general medicines above-mentioned can be dealt with a person having no special qualification such as the pharmacist's license. Further, the first medicines are ordinary medicines that can be dispensed by the dispenser 13. The dispenser 13 previously keeps plural types of the first medicines and dispenses the first medicine to the trays 12 according to requests automatically and mechanically.

The second medicines are special medicines that can be dealt with only by a person having special qualification. Further, the second medicines are not ordinary medicines and held in the medicine rack 16. Large medicines that cannot be kept or dispensed by the dispenser 13 (for example, an infusion pack) are also the second medicines supplied to the trays 12. These second medicines are supplied to the tray 12 by the medicine manager.

The configuration of the medicine collecting system 11 will be described below. The tray 12 has an RFID tag 17, and a tray number is stored in the RFID tag 17. One tray 12 is used to have medicines for one patient, and the tray number is associated to the patient. Thus, the patient can be distinguished, based on the tray number. As a result, when administering a medicine to a certain patient, the doctor reads the tray number in the RFID tag 17 of the tray 12 by means of a tag reader 18 or the like, thereby preventing wrong administration of the medicine for wrong patients.

The dispenser 13 includes the tag reader 18, a touch panel 19, a dispensing unit 20a, a discharging unit 20b, a wireless communication unit 20c and a dispensing processing unit 21.

The tag reader 18 detects the tray number in the RFID tag 17 of the tray 12.

The touch panel 19 accepts an operation input to the dispenser 13 and displays various types of data in the dispenser 13.

The dispensing unit 20a dispenses the first medicines held in the dispenser 13 to the trays 12.

The discharging unit 20b discharges the trays 12, to which the first medicines are dispensed, to the cart 14.

The wireless communication unit 20c sends/receives information between the dispenser 13 and the cart 14.

The dispensing processing unit 21 calculates dispensed medicines on the basis of the prescription information.

The dispensing unit 20a detects names and numbers of dispensed medicines and acquires them as collecting information.

The cart 14 includes the RFID tag 22, the plurality of housing units 23, tray detecting units 24, a wireless communicating unit 25, LEDs (Light Emitting Diode) 26 and a cart processing unit 27.

The RFID tag 22 stores the cart number as an identifier of the cart 14.

Each of the housing units 23 houses one tray 12.

Each of the tray detecting units 24 detects whether or not the tray 12 is located inside each of the housing units 23. The tray detecting unit 24 is, for example, a photoelectric switch provided in each housing unit 23, and the switch may be configured so as to be turned ON when the tray 12 is located at the housing position of the housing unit 23. The tray detecting unit 24 determines that the tray 12 is located at the housing position of the housing unit 23 when the photoelectric switch is turned ON, and determines that the tray 12 is not located at the housing position of the housing unit 23 when the photoelectric switch is turned OFF. Alternatively, the tray detecting unit 24 may be an RFID tag reader provided in each of the housing units 23. In this case, the tag reader should be provided at a position where the tag reader can read data stored in the RFID tag when the RFID tag is adhered to the tray 12 and the tray 12 is located at the housing position of the housing unit 23. Thus, when the tag reader can read data from the RFID tag, the tag reader determines that the tray 12 is located at the housing position of the housing unit 23. On the contrary, when the tag reader cannot read data from the RFID tag, the tag reader determines that the tray 12 is not located at the housing position of the housing unit 23 and is pulled out of the housing unit 23.

The wireless communicating unit 25 transmits/receives information to/from the medicine rack 16.

Each of the LEDs (Light Emitting Diode) 26 is a light emitting unit provided at a position adjacent to each of the housing units 23 and can emit light. The LEDs 26 corresponds to a housing presenting unit in CLAIMS.

The cart processing unit 27 performs control of lighting of the LEDs26 on the basis of the information transmitted from the medicine rack 16 or the detection result of the tray detecting unit 24.

Here, it is assumed that two LEDs 26 is provided at both sides of each housing unit 23 and two LEDs 26 are lighted at the same time for indicating the housing unit 23. However, the LED 26 may be provided at only one side of each housing unit 23.

The managing unit 15 includes the memory unit 28 that stores the medicine collecting list for collecting of necessary medicines. The medicine collecting list contains the housing unit position information (housing unit address) which indicates the position of the housing units 23 housing the trays 12 to which the first medicines are dispensed by the dispenser 13, the prescription information and the dispensing information.

The medicine rack 16 includes a medicine depository 29, the display unit 30, the tag reader 31, a wireless communicating unit 32 and the medicine rack processing unit 33.

The medicine depository 29 holds the second medicines.

The display unit 30 (medicine presenting unit) shows names, numbers and the like of medicines supplied to the tray 12.

The tag reader 31 detects the cart number in the RFID tag 22 of the cart 14.

The wireless communicating unit 32 sends/receives information between the medicine rack 16 and the cart 14.

The medicine rack processing unit 33 (determining unit) calculates the medicines supplied from the medicine rack 16.

With such configuration, when the medicine manager moves the cart 14 to the vicinity of the medicine rack 16, the tag reader 31 of the medicine rack 16 detects the cart number of the cart 14. Then, based on the detected cart number, the medicine rack processing unit 33 of the medicine rack 16 calculates the housing unit address of the housing unit 23 housing the tray 12 that requires supply of the second medicine, and transmits the calculated housing unit address to the cart 14.

Then, the cart 14 lights the LEDs 26, based on the received housing unit address corresponding to the tray 12 that requires supply of the second medicine. When the medicine manager pulls out the tray 12 corresponding to the lighted LEDs 26, the name and the number of the second medicines to be supplied to the tray 12 are shown on the display unit 30 of the medicine rack 16. According to the indication on the display unit 30, the medicine manager supplies the second medicine to the pulled tray 12.

In this manner, the medicine collecting system 11 in accordance with Embodiment 1 can help the medicine manager easily and accurately supply the second medicines to the tray 12 that holds the first medicine dispensed by the dispenser 13 in the state where the tray 12 is pulled out from the carrier cart 14.

Next, a medicine collecting operation using the medicine collecting system 11 will be described in detail.

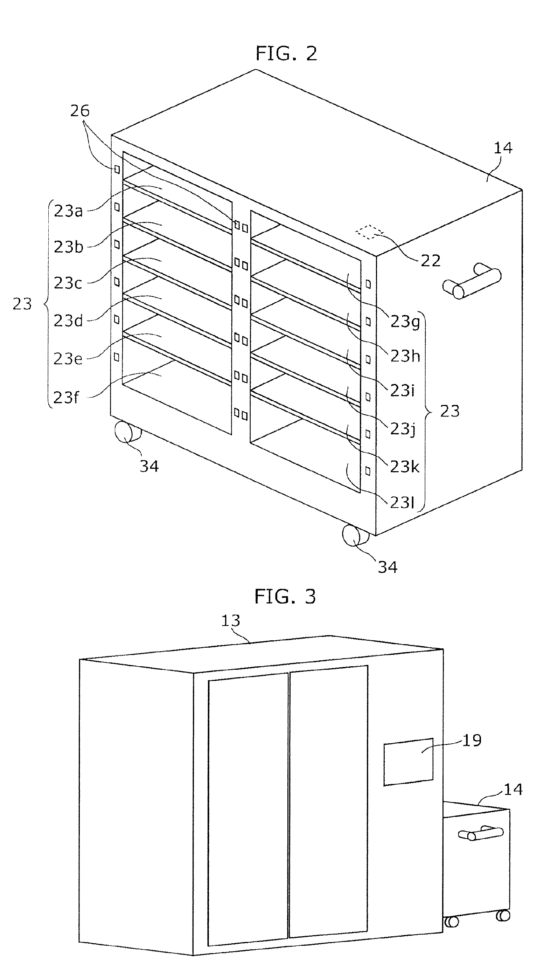

FIG. 2 is a perspective view of the cart 14. The cart 14 has the plurality of housing units 23 (housing units 23a to 231), and can house each of the trays 12 in each of the housing units 23. FIG. 2 shows a vacant state of the trays 12 in the cart 14, where no tray 12 is mounted in the housing units 23 of the cart 14.

The cart 14 further includes the LEDs 26 as light emitting units adjacent to both sides of a housing port of each housing unit 23. The cart 14 includes wheels 34 as means for movement.

The housing unit address is assigned to each of the housing units 23, the housing unit address of the housing unit 23a is A1, the housing unit address of the housing unit 23b is A2, the housing unit address of the housing unit 23c is A3, the housing unit address of the housing unit 23d is A4, the housing unit address of the housing unit 23e is A5, the housing unit address of the housing unit 23f is A6, the housing unit address of the housing unit 23g is B1, the housing unit address of the housing unit 23h is B2, the housing unit address of the housing unit 23i is B3, the housing unit address of the housing unit 23j is B4, the housing unit address of the housing unit 23k is B5 and the housing unit address of the housing unit 231 is B6.

One housing unit 23 has two LEDs 26.

First, the medicine manager who performs the medicine collecting operation moves the vacant cart 14 that contains no tray 12 to the dispenser 13, and couples a tray gate of the cart 14 to a dispensing port of the dispenser 13.

FIG. 3 is a view showing the cart 14 and the dispenser 13 in the state where the tray gate of the cart 14 is coupled to the dispensing port of the dispenser 13 at dispensing of medicines by the dispenser 13.

The dispenser 13 automatically dispenses the first medicine to the vacant tray 12 according to the prescription information. The dispenser 13 houses the tray 12 to which the first medicine is dispensed in the housing unit 23 of the cart 14. The dispenser 13 is operated with the touch panel 19 by the medicine manager.

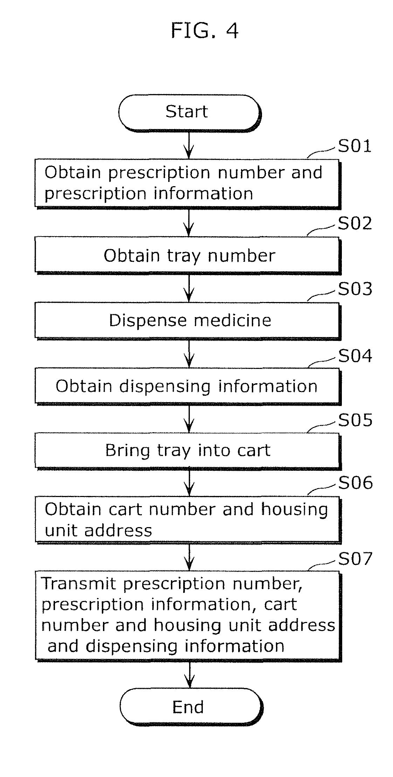

Next, details of the operation of the dispenser 13 will be described using a flow chart in FIG. 4. When the medicine manager pushes a medicine dispensing button of the touch panel 19 of the dispenser 13 after the cart 14 is coupled to the dispenser 13, the dispenser 13 starts dispensing of first medicines required among various first medicines, which are previously housed in the dispenser 13.

First, the dispensing processing unit 21 acquires the prescription number and the prescription information from the medicine collecting list stored in the managing unit 15 through the wireless communication unit 20c (Step S01).

The prescription information represents the type and the number (dose) of the medicines, and the prescription number is used for managing the prescription information.

Next, the dispensing processing unit 21 acquires the tray number of the tray 12 through the tag reader 18 (Step S02).

The dispensing processing unit 21 dispenses the first medicine from the dispensing unit 20a to the tray 12 according to the prescription information (Step S03). That is, the dispensing processing unit 21 dispenses the first medicines among a plurality of medicines in the dispenser 13, which are contained in the prescription information.

The dispensing processing unit 21 acquires the dispensing information, representing the name and the number (dose) of the first medicines dispensed from the dispensing unit 20a (Step S04).

Next, the discharging unit 20b houses the tray 12, to which the first medicine is dispensed, into the housing unit 23 of the cart 14 (Step S05).

At this time, the dispensing processing unit 21 acquires, from the cart 14, the cart number of the cart 14 into which the tray 12 is housed and the housing unit address of the housing unit 23 that houses the tray 12 (Step S06). That is, the dispensing processing unit 21 acquires the cart number from the RFID tag 22 of the cart 14 through the tag reader 18. The cart 14 includes the tray detecting unit 24 that detects whether or not the tray 12 is located at the housing position of each housing unit 23. When detecting that the tray 12 is located at the housing position, the tray detecting unit 24 determines that the tray 12 is housed into the housing unit 23 of the cart 14 from the dispenser 13. In response to bringing-in of the tray 12, the wireless communicating unit 25 transmits the housing unit address of the housing unit 23, into which the tray 12 is housed, to the wireless communication unit 20c of the dispenser 13. The dispensing processing unit 21 acquires the housing unit address of the housing unit 23, into which the tray 12 is brought, from the wireless communication unit 20c.

Then, the dispensing processing unit 21 transmits the cart number and the housing unit address, which are acquired in Step S06, the dispensing information representing information on the medicine dispensed to the tray 12, the prescription information and the prescription number to the managing unit 15 (Step S07). The dispensing processing unit 21 also transmits the tray number to the managing unit 15 as needed.

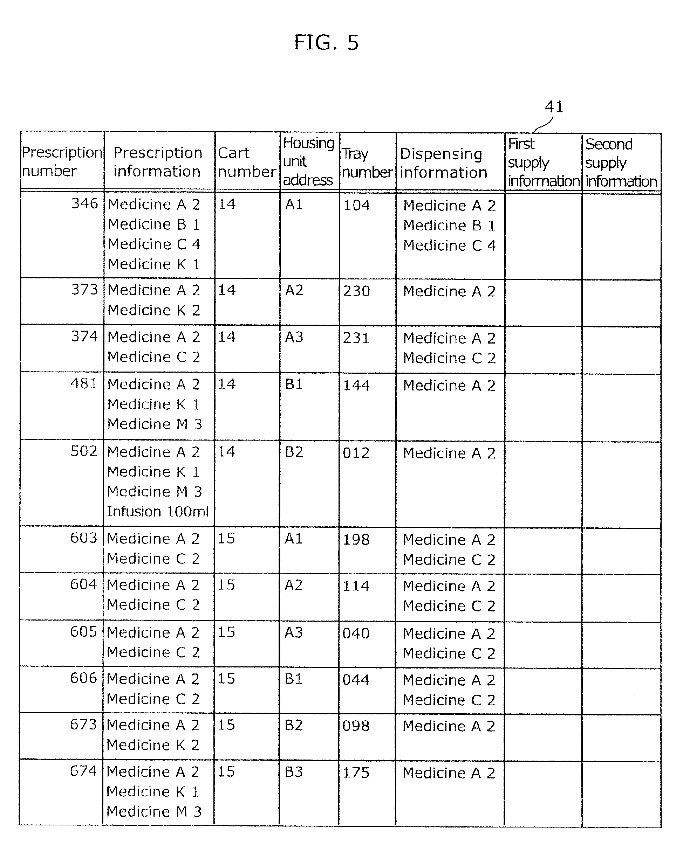

The managing unit 15 writes the cart number, the housing unit address, the dispensing information, the prescription number and the prescription information, which are received from the dispensing processing unit 21 of the dispenser 13, as the medicine collecting list into the memory unit 28. FIG. 5 is a view showing an example of a medicine collecting list 41. For example, the list shows that, as to prescription expressed by the prescription number "346", names and the number of the medicines to be prescribed are "two medicines A" "one medicine B1", "four medicines C" and "one medicine K", and among them, the "two medicines A", the "one medicine B1" and the "four medicines C" are collected as the first medicines to the tray 12 having the tray number "104". The list also shows that the tray 12 is housed at the housing unit address "A1" of the housing unit 23 (housing unit 23a) of the cart 14 having the cart number "14". The tray number corresponding to the prescription number and the prescription information is written to the medicine collecting list as needed.

In this manner, the dispenser 13 repeats the operation shown in flow chart in FIG. 4 the required number of times to discharge the plurality of the trays 12 that hold the medicines dispensed by the dispenser 13 to the plurality of the housing units 23 of the cart 14.

After the dispenser 13 discharges the required number of trays 12 to the cart 14, the medicine manager moves the cart 14 to the vicinity of the medicine rack 16 to supply the second medicines to the cart 14.

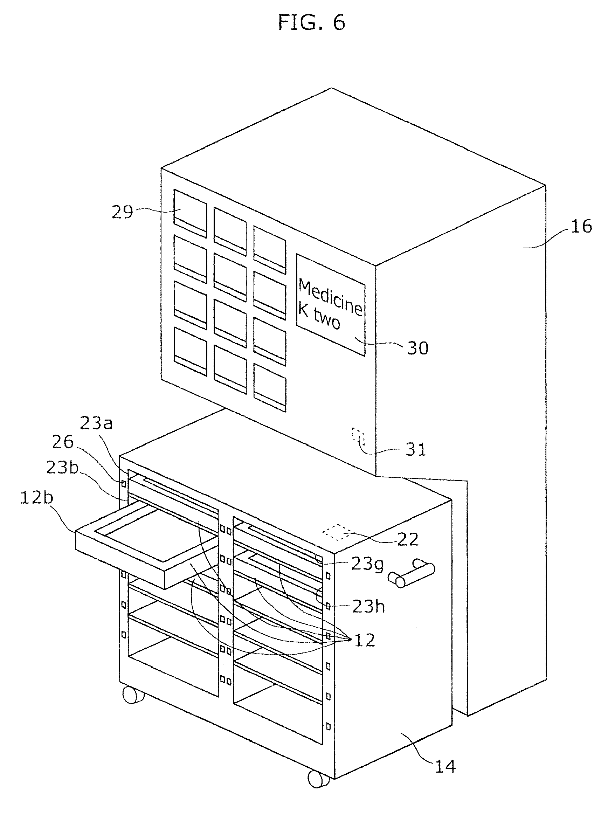

FIG. 6 is a view showing the medicine rack 16 and the cart 14, where the cart 14 is moved to the vicinity of the medicine rack 16 to supply the medicine.

The medicine manager pulls the tray 12 out of the cart 14 and supplies the second medicine to the tray 12. The state where the tray 12 is pulled out of the cart 14 does not mean the state where the tray 12 is wholly taken out of the cart 14 but the state of the tray 12b shown in FIG. 6, that is, the state where a part of the tray 12b is left in the housing unit 23b and can receive a medicine.

When the tray 12b in the housing unit 23b with the lighted LEDs 26 is pulled out, the names and the numbers of the second medicines to be supplied to the tray 12b are shown on the display unit 30. The medicine manager supplies the second medicine according to the name and the number of the medicines.

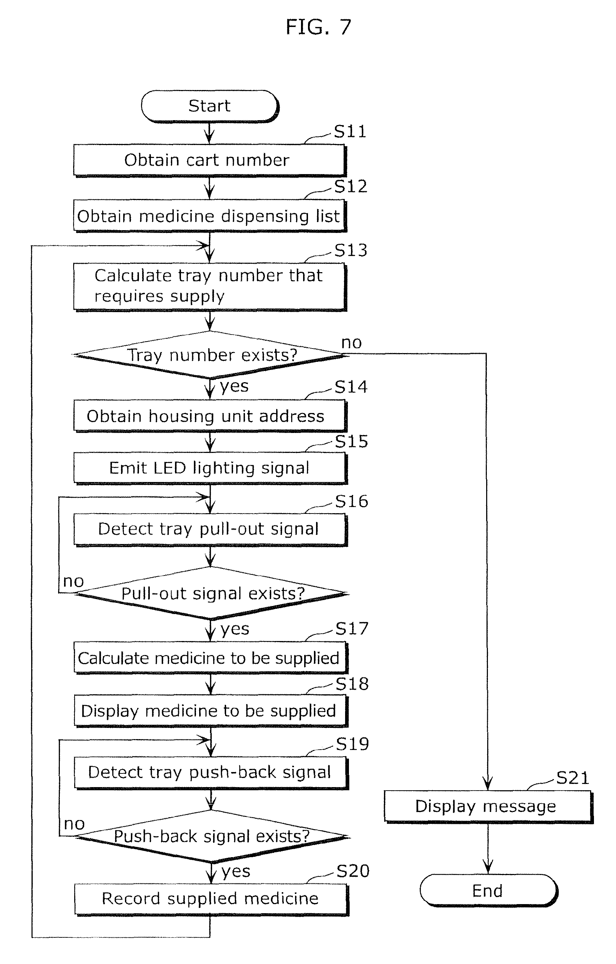

Next, the operation of the medicine rack 16 will be described using the flow chart in FIG. 7.

First, the medicine rack processing unit 33 detects the cart number from the RFID tag 22 of the cart 14 through the tag reader 31. When the cart 14 enters into a detection area within one meter radius of the tag reader 31 of the medicine rack 16 (hereinafter referred to as "medicine rack operating area"), the medicine rack 16 reads information in the RFID tag 22 of the cart 14 by wireless communication and acquires the cart number of the cart 14 (Step S11). The medicine rack 16 executes processing in steps following Step S12 when the cart number can be acquired, and does not execute the processing in steps following Step S12 when the cart number cannot be acquired. The medicine rack operating area is not limited to the above-mentioned area and may be freely set by the operator of the medicine collecting system 11.

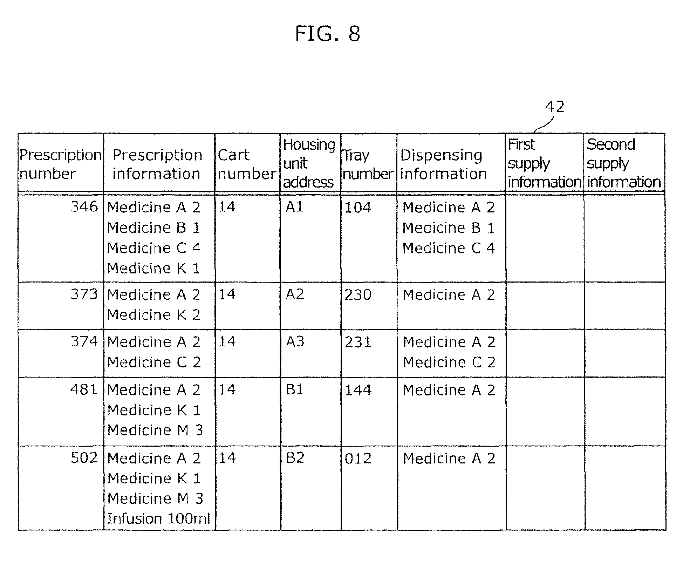

In order to acquire the collecting list for the detected cart number, the medicine rack processing unit 33 transmits the detected cart number to the managing unit 15, and acquires a collecting list 42 for the detected cart number as shown in FIG. 8 (Step S12).

The medicine rack processing unit 33 calculates the tray number that needs to be supplied the medicines according to the acquired collecting list 42 (Step S13).

Describing Step S13 in detail, medicines that can be supplied from the medicine rack 16 is the second medicines kept in the medicine depository 29. In the collecting list 42 shown in FIG. 8, the prescription information of the prescription numbers "346", "373", "481" and "502" contains a medicine K or a medicine M as the second medicine kept in the medicine rack 16. Thus, the trays 12 to which the medicine K or the medicine M is collected have the tray numbers "104", "230", "144" and "012".

The medicine rack processing unit 33 acquires the housing unit addresses of the housing units 23 in which the tray 12 having these tray numbers are housed (Step S14). In the above-mentioned example, the housing unit addresses "A1", "A2", "B1" and "B2" are acquired. That is, through the operations of the medicine rack processing unit 33 in Step S13 and Step S14 (operations of the determining unit), based on the prescription information in the collecting list corresponding to the cart, presence or absence of the second medicine to be supplied to each tray in the cart is checked, and when the second medicine exists, the housing unit address (housing unit identification information) of the cart in which the tray corresponding to the second medicine is housed is determined according to the prescription information.

The medicine rack processing unit 33 generates a lighting signal containing the housing unit address acquired in Step S14 (in the above-mentioned example, the housing unit addresses "A1", "A2", "B1" and "B2") and transmits the lighting signal to the cart 14. In this manner, the medicine rack processing unit 33 instructs the cart 14 to light the LEDs 26 of the housing unit 23 that houses the tray 12 requiring supply of the second medicine (Step S15).

Then, the cart processing unit 27 lights the LEDs 26 corresponding to the housing unit 23 having the housing unit address (in the above-mentioned example, the housing unit addresses "A1", "A2", "B1" and "B2") contained in the received lighting signal. That is, in the above-mentioned example, as shown in FIG. 6, the cart processing unit 27 lights the LEDs 26 located on both sides of the housing units 23a, 23b, 23g and 23h. That is, the operation of the medicine rack processing unit 33 in Step S15 and the operation of the LEDs 26 (operation of the housing presenting unit) present information for identifying the cart housing unit represented by the housing unit address (housing unit identification information) determined in Step S14. The cart processing unit 27 may present the housing unit by allowing the LEDs 26 to flash in place of lighting the LEDs 26.

The medicine manager can find the tray 12 that requires supply by viewing lighting of the LEDs 26, and pulls out one of the trays 12 of the plurality of housing units with the lighted LEDs 26.

FIG. 6 shows the state where the tray 12b having the tray number "230" is pulled out from the housing unit 23b.

The cart 14 includes the tray detecting unit 24 that detects whether or not the tray 12 is located at the housing position of the housing unit 23. The state where the tray 12 is located at the housing position means the state where the tray 12 is wholly housed in the housing units 23. The state where the tray 12 is not located at the housing position means the state where the tray 12 is pulled out of the housing units 23 for receiving a medicine.

In the cart 14 shown in FIG. 6, the tray 12 in the housing unit 23b is not located at the housing position, and the trays 12 in the housing units 23a, 23g and 23h are located at the housing position.

When the tray detecting unit 24 detects that the tray 12 at the housing position does not exist, that is, when the tray 12 is pulled out, the cart processing unit 27 transmits a pull-out signal, containing the housing unit address of the housing unit 23 with the pulled tray 12 (for example, the housing unit address "A2" of the housing unit 23b), to the medicine rack 16 via the wireless communicating unit 25.

The medicine rack processing unit 33 of the medicine rack 16 detects the pull-out signal transmitted from the cart 14 via the wireless communicating unit 32 (Step S16).

When detecting the pull-out signal, the medicine rack processing unit 33 of the medicine rack 16 calculates second medicines to be supplied to the pulled tray 12 (Step S17). That is, the medicine rack processing unit 33 calculates the medicine in the medicine rack 16 among the medicines that are not dispensed to the pulled tray 12, as the medicine to be supplied.

For example, the housing unit address contained in the pull-out signal is "A2", and based on the housing unit address"A2", the medicine rack processing unit 33 calculates the medicine, to be supplied, according to the collecting list 42. That is, as the medicines to be supplied to the tray 12 having the tray number "230", which is housed at the housing unit address "A2", "two medicines K2" are calculated.

The medicine rack processing unit 33 displays the name and the number of the calculated medicine to be supplied on the display unit 30 (Step S18). That is, the display unit 30 in FIG. 6 shows the medicine K and two as the name and the number of the medicine to be supplied, respectively.

When the medicine manager pulls out the tray 12 with the unlighted LEDs 26, the name and the number of the medicine to be supplied cannot be displayed and therefore, an alarm may be sounded to issue a warning to the medicine manager.

The medicine manager takes out the medicine to be supplied from the medicine depository 29 according to the display on the display unit 30 and collects the medicine to the tray 12.

When collecting of the medicine to be supplied is finished, the medicine manager pushed back the tray 12 to the housing unit 23, thereby housing the tray 12 in the housing unit 23.

When the tray detecting unit 24 newly detects the tray 12 that did not exist at the housing position, the cart processing unit 27 recognizes that the tray 12 is pushed back to the housing unit 23 and generates a push-back signal containing the housing unit address of the housing unit 23 pushed back. Then, the cart processing unit 27 transmits the push-back signal from the cart 14 to the medicine rack 16 via the wireless communicating unit 25.

While the medicine manager collects the medicine to be supplied, the medicine rack processing unit 33 of the medicine rack 16 detects presence or absence of the push-back signal (Step S19). When detecting the push-back signal, the medicine rack processing unit 33 recognizes that the supply of the medicine to the tray 12 is completed.

Then, the medicine rack processing unit 33 enters the name and the number of the medicine in a column for first supply information in the collecting list 42, shown in FIG. 8, to record the supplied medicine (Step S20).

When finishing recording of the supplied medicine in Step S20, the medicine rack processing unit 33 returns to Step S13 and calculates the tray number of the tray 12 that requires supply of a medicine.

As to the tray 12, to which the medicine has been already supplied, the supplied medicine is recorded in the column for first supply information in the collecting list 42. For this reason, in subsequent processing, there is no possibility that the tray 12, to which the medicine has been already supplied, is calculated as the tray 12 that requires supply of a medicine from the medicine rack 16.

The medicine rack 16 repeats Steps S13 to S20 so that the medicine manager pulls out the tray 12 housed in the housing unit with the lighted LEDs 26 and supplies a medicine to the tray 12.

When supply of medicines from the medicine rack 16 to all of the trays that require supply of medicines is finished and the tray number of the tray that requires supply cannot be calculated, the display unit 30 of the medicine rack 16 displays a message that supply of medicines to the cart 14 is completed (Step S21).

As described above, in Embodiment 1, the medicine collecting system 11 can easily find the tray 12 that requires supply of the second medicine, even when the tray 12 is mounted in the carrier cart 14.

The cart 14 includes the tray detecting unit 24 that detects whether or not the tray 12 housed in the housing unit 23 is pulled out. When the tray detecting unit 24 detects that the tray 12 is pulled out, the cart 14 transmits the pull-out signal containing the housing unit address of the pulled housing unit 23 to the medicine rack 16. Thereby, the medicine rack 16 can display the name and the number of the second medicines supplied to the pulled tray 12 on the display unit 30. Accordingly, the medicine manager can easily supply the medicine according to display on the display unit 30.

The infusion pack, so large that it cannot be kept in the medicine rack 16, a temperature-controlled medicine, requiring temperature control, and so on may be kept in another medicine depository. Such medicine depository, if any, may be used in cooperation with the medicine collecting system 11.

For example, as shown in the collecting list 42 in FIG. 8, the tray 12 having the tray number "012" needs to be supplied an infusion pack of 100ml that cannot be supplied from the medicine rack 16. In this case, the medicine manager supplies the medicines K, M from the medicine rack 16 and then, moves the cart 14 to the medicine depository that keeps the infusion pack. The medicine depository detects the cart number of the cart 14. Like the medicine rack 16, the medicine depository calculates the tray 12 that requires supply of the medicine kept in the medicine depository on the basis of the cart number, and causes the cart 14 to light the LEDs 26 of the housing unit 23 that houses the tray 12. Thereby, the medicine depository can inform the medicine manager of the tray 12 to which the infusion pack is supplied. The medicine depository enters the name of the medicine to be supplied and the number of the medicines into a column for second supply information in the collecting list 42 in FIG. 8 to record the medicine to be supplied.

The medicine rack 16 displays the medicine to be supplied in Step S18, and in addition, may be configured to unlock only the medicine depository that keeps the displayed medicine or to automatically pop up only the medicine depository that keeps the displayed medicine. This enables the medicine manager to avoid wrong selection of the medicine to be supplied and collect the correct medicine.

Further, the medicine rack 16 may be provided with a medicine detecting unit such as a bar-code reader. The medicine taken from the medicine rack 16 may be detected by the medicine detecting unit, and after check of the medicine name, the medicine may be discharged to the tray 12. That is, the medicine manager reads a bar code assigned to the medicine supplied to the tray 12 by means of the medicine detecting unit. At Step S20, the medicine rack processing unit 33 enters the name and the number of the medicines read by the medicine detecting unit, in place of entering the name and the number of the medicines that requires supply, in the column for the first supply information in the collecting list 42 in FIG. 8, to record the detected correct medicine to be supplied. This also enables the medicine manager to avoid wrong selection of the medicine and collect the medicine more correctly. In place of reading of the bar code, an RFID tag that records the medicine name thereon may be adhered to the medicine or its package and the medicine name recorded on the RFID tag may be read by means of a tag reader.

### (Embodiment 2)

The medicine collecting system 11 in accordance with Embodiment 1 includes the medicine rack, while a medicine collecting system in accordance with Embodiment 2 of the present invention does not include the medicine rack. In Embodiment 2, a carrier cart, that houses the trays holding the first medicines dispensed by a dispenser, is provided with a medicine detecting unit (for example, a bar-code reader) for detecting medicines. When the medicine detecting unit detects a code or name of the medicine, the medicine collecting system in accordance with Embodiment 2 informs the medicine manager of the tray that requires supply of the medicine having the code or name detected by the medicine detecting unit.

By using the medicine collecting system 61 in accordance with Embodiment 2, the medicine manager can easily and correctly supply the medicine to the tray that holds the medicine dispensed by the dispenser.

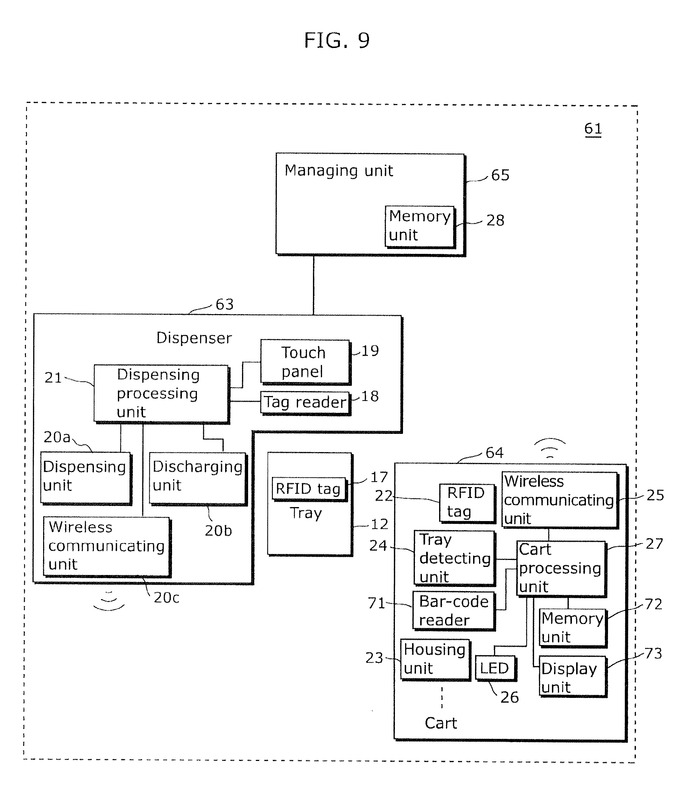

FIG. 9 is a functional block diagram showing a configuration of a medicine collecting system 61 in accordance with Embodiment 2.

The medicine collecting system 61 includes a dispenser 63, a cart 64 and a managing unit 65.

The dispenser 63 dispenses the first medicines to the trays 12 according to the prescription information, and houses each of the plurality of the trays 12 dispensed in each of the housing units 23 of the cart 64, respectively. Here, the prescription information is information indicating the type and the number of the medicines to be dispensed to the tray 12, and each prescription information is associated with each of the trays 12 to which the medicines are dispensed. The dispenser 63 has the same configuration as the dispenser 13 shown in FIG. 1.

The cart 64 includes a bar-code reader 71, a memory unit 72 and a display unit 73, in addition to the constituents of the cart 14 shown in FIG. 1. The bar-code reader 71 is a medicine detecting unit that detects the medicine name from the medicine code. The memory unit 72 stores the medicine collecting list and the like. The display unit 73 as the medicine presenting unit displays the name, the number and so on of the medicine to be supplied to the tray 12.

When the bar-code reader 71 detects the name of the second medicine, the cart processing unit 27 (determining unit) calculates the housing unit address of the housing unit 23 that houses the tray 12 requiring supply of the medicine detected by the bar-code reader 71. The housing position of the tray 12, which is calculated by the cart processing unit 27, is shown on the display unit 73 to inform the medicine manager of the housing position of the tray 12 that requires supply.

That is, when calculating the housing unit address of the housing unit 23 that requires supply of the detected second medicine, the cart processing unit 27 lights the LEDs 26 indicating the tray 12, requiring supply of the medicine, according to the calculated housing unit address. When the medicine manager pulls out the tray 12 corresponding to the lighted LEDs 26, the name and the number of the medicines to be supplied to the pulled tray 12 are displayed on the display unit 73 of the cart 64. According to the display, the medicine manager supplies the medicine to the tray 12.

In this manner, the medicine manager can easily and correctly supply the medicine to the tray, which holds the medicine dispensed by the dispenser 63 and is pulled out from the cart 64.

The managing unit 65 includes the memory unit 28. The memory unit 28 stores the prescription information, the dispensing information indicating the type and the number of the first medicine dispensed to the tray 12 by the dispenser 63 according to the prescription information and the housing unit position information (housing unit address) indicating the position of the housing unit 23 in the cart, which houses the tray 12 to which the first medicine is dispensed. That is, the managing unit 65 includes the memory unit 28 that stores the medicine collecting list necessary for collecting the medicines. The collecting list contains the prescription information and the dispensing information, which are associated with the housing unit position information (housing unit address) indicating the position of the housing unit 23 that houses the tray 12 to which the first medicine is dispensed. The collecting list held in the memory unit 28 of the managing unit 65 is accessed by the dispensing processing unit 21 of the dispenser 63 as appropriate. That is, the dispensing processing unit 21 of the dispenser 63 can record information into the collecting list in the memory unit 28 of the managing unit 65 and obtains information from the collecting list.

Next, a medicine collecting operation using the medicine collecting system 61 will be described in detail.

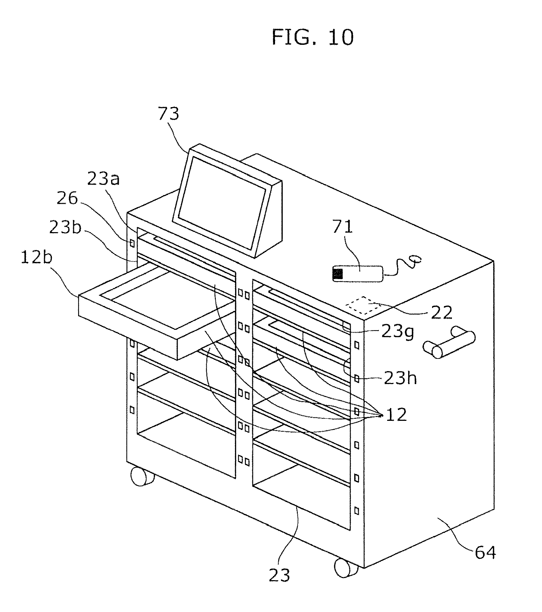

FIG. 10 is a perspective view of the cart 64. The cart 64 has the plurality of the housing units 23 (for example, the housing units 23a, 23b, 23g, 23h) and houses the trays 12 in the respective housing units 23. In the cart 64 shown in FIG. 10, the trays 12 are brought into the housing units 23a, 23b, 23g and 23h from the dispenser 63.

The cart 64 includes the display unit 73, for displaying the name and the number of the medicines, and the bar-code reader 71, for reading the bar code of the medicine to detect the name of the medicine.

First, the medicine manager who performs the medicine collecting operation moves the vacant cart 64 that does not mount the tray 12 to the dispenser 63 and couples a tray gate of the cart 64 to a discharge port of the dispenser 63.

The dispenser 63 automatically dispenses the first medicine to the vacant tray 12 according to the prescription information. Further, the dispenser 63 houses the tray 12, to which the first medicine is dispensed, to the housing unit 23 of the cart 64. The medicine manager operates the dispenser 63 by means of the touch panel 19.

The operations of the dispenser 63, dispensing the first medicine to the tray 12 and housing the tray 12 in the housing unit of the cart 64, are the same as Steps S01 to S07 in Embodiment 1. Thus, detailed description of the operations is omitted.

When dispensing of the medicines from the dispenser 63 to the plurality of trays is finished, before separation of the cart 64 from the dispenser 63, the collecting list is transmitted from the wireless communication unit 20c of the dispenser 63 to the wireless communicating unit 25 of the cart 64. That is, the dispensing processing unit 21 of the dispenser 63 acquires the collecting list from the memory unit 28 of the managing unit 65, and transmits the collecting list, corresponding to the cart number of the coupled cart 64, to the cart processing unit 27 of the coupled cart 64. For example, when the cart number of the coupled cart 64 is "14", the collecting list of the cart number "14", as shown in FIG. 8, is transmitted. The cart processing unit 27 writes the received collecting list 42 into the memory unit 72 of the cart 64.

The medicine manager moves the cart 64 having the plurality of the trays 12 to a medicine room located away from the dispenser 63, and supplies the second medicines in the medicine room.

Information on the name and the number of the medicines to be supplied is transmitted from the managing unit 65 to a management terminal (not shown) in the medicine room, and the information is displayed on a display screen of the management terminal. That is, information on the name and the number of the medicines, which are not yet collected (that is, the medicines to be supplied) among the medicines in the prescription information in the collecting list corresponding to the cart number of the cart 64, is displayed on the display screen of the management terminal. Thus, the medicines to be supplied to the cart 64 can be previously prepared in the medicine room. Since it is burdensome to prepare the medicines for each tray 12, the medicines to be supplied for the cart 64 are collectively prepared.

For example, in the collecting list of the cart number "14" as shown in FIG. 8, the name and the total number of the medicines to be supplied are "medicine K, five", "medicine M six" and "infusion, 100 ml". The information may be displayed on the display unit 73 of the cart 64, not on a display unit of the management terminal.

The medicine manager brings the prepared medicines to be supplied together to the cart 64 and collects the medicines to the trays 12 of the cart 64, which require supply of the medicines.

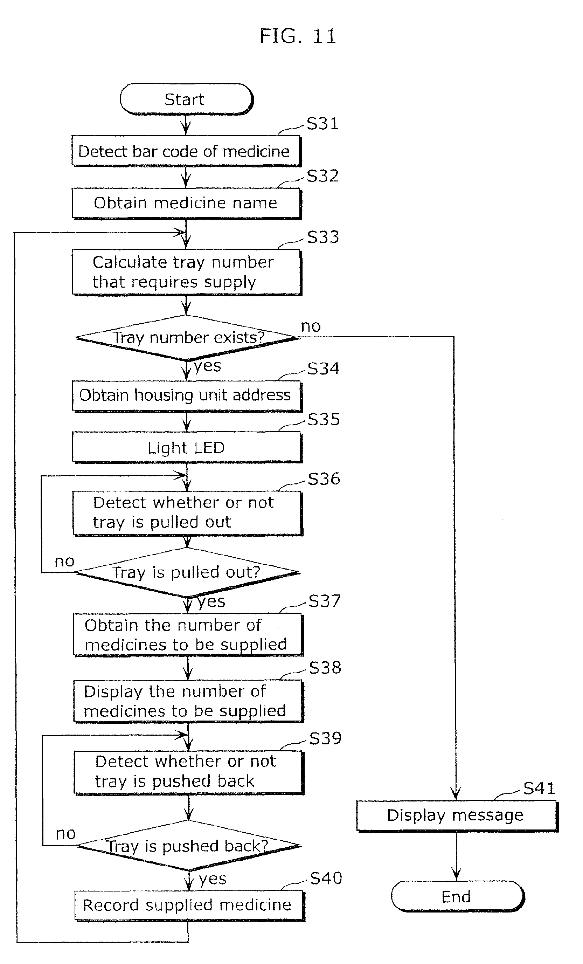

Next, details of the supply operation to the cart 64 will be described, using a flow chart in FIG. 11.

The medicine manager moves the bar code of a medicine closer to the bar-code reader 71. Thereby, the bar-code reader 71 of the cart 64 detects the bar code of one medicine (one type of medicine) (Step S31), and acquires the medicine name on the basis of the detected bar code information (Step S32).

The cart processing unit 27 compares the medicine name acquired in Step S32 with the prescription information in the collecting list 42 stored in the memory unit 72, thereby calculating the tray number of the tray 12 that requires supply of this type of the medicine (Step S33). Here, the medicine name is stored in the prescription information, and the medicine name in the prescription information is compared with the detected medicine name. However, the medicine code may be stored in the prescription information, and the medicine code in the prescription information may be compared with the detected medicine bar code.

Providing that the medicine name detected in Step S32 is the medicine K. The prescription information that contains the medicine K in the collecting list 42 is the prescription information on the tray numbers "104", "230", "144" and "012". So the tray numbers of the trays 12 that require supply of the medicine K are "104", "230", "144" and "012".

Referring to the collecting list 42, the cart processing unit 27 acquires the housing unit addresses "A1", "A2", "B1" and "B2" corresponding to the tray numbers "104", "230", "144" and "012" (Step S34). That is, through the operations of the cart processing unit 27 in Step S33 and Step S34 (operations of the determining unit), the presence or absence of the second medicine to be supplied to each tray of the cart is checked on the basis of the prescription information in the collecting list corresponding to the concerned cart. When the second medicine exists, the housing unit address (housing unit identification information) of the tray in the cart corresponding to the second medicine is determined according to the prescription information.

The cart processing unit 27, as shown in FIG. 10, lights the LEDs 26 of the housing units 23a, 23b, 23g and 23h corresponding to the housing unit addresses "A1", "A2", "B1" and "B2" acquired in Step S34 (Step S35). That is, the operation of the cart processing unit 27 in Step S35 and the operation of the LEDs 26 (operations of the housing presenting unit) present information to identify the housing unit of the cart, which is indicated by the housing unit address (housing unit identification information) determined in Step S34.

The medicine manager can find the trays 12 that require supply by viewing lighting of the LEDs 26, and pulls out one tray 12 among the plurality of housing units with the lighted LEDs 26.

FIG. 10 is a view showing the state where the tray 12b, having the tray number "230" in the housing units 23b, is pulled out.

In the cart 64 shown in FIG. 10, the tray 12 in the housing unit 23b is not located at the housing position (pulled state), the trays 12 in the housing units 23a, 23g and 23h are located at the housing position (non-pulled state).

The tray detecting unit 24 detects presence or absence of pulling-out of the tray 12 (Step S36).

When the tray detecting unit 24 detects pulling-out of the tray 12, the cart processing unit 27 acquires information on the number of the medicines to be supplied to the tray 12 (Step S37). For example, in the case where the medicine name detected by the bar-code reader 71 is the medicine K and the housing unit address of the pulled tray 12 is "A2", the cart processing unit 27 acquires the number of medicines K from the collecting list 42, supplied to the tray 12, on the basis of the housing unit address "A2" and the medicine name "medicine K". Here, the number of the medicines K to the tray 12 at the housing unit address "A2" is two.

According to an instruction from the cart processing unit 27, the number of the second medicines to be supplied is displayed on the display unit 73 of the cart 64 (Step S38). Here, two as the number of the medicines K is displayed. According to the display on the display unit 73, the medicine manager supplies the number of the second medicines to the tray 12.

When collecting of the supplied second medicine (medicine K) is finished, the medicine manager pushes back the tray 12 into the housing units 23.

When the tray detecting unit 24 of the cart 64 detects that the tray 12 is located at the housing position of the housing unit 23, the cart processing unit 27 recognizes that the tray 12 is pushed back to the housing units 23.

While the medicine manager collects the medicines, the cart processing unit 27 detects whether or not the tray 12 is pushed back (Step S39). When detecting that the tray 12 is pushed back, the cart processing unit 27 recognizes that supply of the medicine (medicine K) to the pulled tray 12 is completed.

Then, the cart processing unit 27 records the name and the number of the medicines in the column for the first supply information in the collecting list 42 in the memory unit 72 in FIG. 8 (Step S40).

When finishing recording of the medicines to be supplied in Step S40, the cart processing unit 27 returns to Step S33 and calculates the tray number of the tray 12 that requires supply of the same type of the medicine.

In this stage, as to the tray 12 having the tray number, to which the medicine is supplied, the supplied medicine is recorded in the column for the first supply information in the collecting list 42 in the memory unit 72. For this reason, in subsequent processing, the tray 12 having the tray number, to which the medicine is supplied, is not calculated as the tray 12 that requires supply of the same medicine acquired in S32.

The cart 64 repeats Step S33 to S40, and the medicine manager pulls out the tray 12 in the housing unit 23 with the lighted LEDs 26 and supplies the medicine (medicine K) to the tray 12.

When supply of the medicine having the medicine name detected by the bar-code reader 71 (medicine K) to the tray 12 is finished and the tray number that requires supply is no longer calculated in Step S33, the display unit 73 displays a message, representing that supply of the medicine having the medicine name (medicine K) is finished (Step S41).

Next, the medicine manager moves the bar-code reader 71 closer to another type of the medicine (for example, the medicine M). Thereby, the bar-code reader 71 acquires the medicine name of another type of the medicine. After that, the cart 64 performs the same steps as described above, so that the medicine manager supplies the medicine (medicine M) to the tray 12 of the cart 64.

When the bar-code reader 71 of the cart 64 detects the name of the medicine to be supplied, the LEDs 26 corresponding to the housing unit 23 housing the tray 12 that requires supply of the medicine having the detected medicine name are lighted. Thus, the medicine manager can easily find the tray 12 that requires supply of the medicine. In turn, the medicine manager can easily and correctly supply the second medicine.

Since the display unit 73 displays the type and the number of the second medicine, the medicine manager can easily and correctly supply the second medicine.

Although the medicine collecting system in accordance with the embodiments of the present invention has been described, the present invention is not limited to these embodiments.

For example, although the LED is used as the housing presenting unit that presents the housing unit housing the tray that requires supply of the medicine in the above-mentioned embodiments, another display such as a liquid crystal display may present the housing unit. Alternatively, the housing presenting unit as the display unit may display information on the position of the housing unit housing the tray that requires supply of the medicine. The housing presenting unit may present an identifier such as a number previously assigned to each housing unit to the medicine manager. The positional information of the housing unit may be notified to the medicine manager by sound.

Although it is assumed that the medicine manager pulls one tray 12 out of the housing units 23 of the cart in the above-mentioned embodiments, the medicine manager may pull the plurality of trays 12 out of the housing units 23. Information on the housing position of the tray 12 may be associated with information on the medicine to be supplied, and the associated information may be displayed on the housing presenting unit.

The tray in the above-mentioned embodiment is suitable for holding medicines and mounting in the cart. However, an object having the same function as the tray (for example, a medicine pouch that contains a medicine) falls within the scope of the present invention.

It is to be understood that the embodiments disclosed above are intended not for the purpose of limitation but for exemplification only in all respects. The scope of this invention is indicated not by the aforementioned description but by the Claims, and it is intended that various changes and modifications within the same or equivalent meaning and scope of the Claims may be included.

### [Industrial Applicability]

The medicine collecting system according to the present invention can easily supply the medicine to the correct tray among the plurality of the trays in the cart, each of which holds the medicines and therefore, and is useful as the medicine collecting system installed in a hospital or a similar facility.

### [Reference Signs List]

- 11, 61: medicine collecting system
- 12: tray
- 13, 63: dispenser
- 14, 64: cart
- 15, 65: managing unit
- 16: medicine rack
- 17, 22: RFID tag
- 18, 31: tag reader
- 19: touch panel
- 20a: dispensing unit
- 20b: discharging unit
- 21: dispensing processing unit
- 23: housing unit
- 24: tray detecting unit
- 20c, 25, 32: wireless communicating unit
- 26: LED
- 27: cart processing unit
- 28, 72: memory unit
- 29: medicine depository
- 30, 73: display unit
- 33: medicine rack processing unit
- 34: wheel
- 41, 42: collecting list
- 71: bar-code reader

## Claims

1. A medicine collecting system that assists in collecting of medicines, the system comprising:
a cart that includes a plurality of housing units each housing a tray to which a first medicine is dispensed according to prescription information;
a memory unit configured to store, for each tray, cart information that identifies the cart, the prescription information and housing unit identification information that identifies the housing unit housing the tray to which the first medicine is dispensed, in association with one another;
a determining unit configured to check, from information of the memory unit, for presence or absence of a second medicine to be supplied to each tray housed in each housing unit of the cart identified on the basis of the cart information, and determine, according to the prescription information, the housing unit identification information which identifies the housing unit needed to contain the second medicine; and
a housing presenting unit configured to indicate the housing unit identified by the housing unit identification information determined by the determining unit.

2. The medicine collecting system according to Claim 1, further comprising
a dispenser that automatically dispenses the first medicine to each tray according to the prescription information set for the tray.

3. The medicine collecting system according to Claim 1, further comprising a medicine detecting unit configured to detect a code of a medicine,
wherein the determining unit configured to check for presence or absence of the second medicine on the basis of the code detected by the medicine detecting unit.

4. The medicine collecting system according to Claim 1, wherein
the housing presenting unit is provided in the cart, and
the medicine collecting system further comprises:
a light emitting unit provided for each of the housing units so as to be at a position corresponding to the housing unit; and
a cart processing unit configured to light the light emitting unit corresponding to the housing unit identified by the housing unit identification information determined by the determining unit, to present information that indicates the housing unit identified by the housing unit identification information determined by the determining unit.

5. The medicine collecting system according to Claim 1, further comprising
a medicine presenting unit configured to present a type of the second medicine and the number of the second medicines determined on the basis of the prescription information and the first medicine, for each tray.

6. The medicine collecting system according to Claim 1, further comprising
a medicine rack that keeps the second medicine and includes the medicine presenting unit.

7. The medicine collecting system according to Claim 6,
wherein a medicine rack operating area is set around the medicine rack, and
the housing presenting unit is configured to present the information that indicates the housing unit identified by the housing unit identification information determined by the determining unit only when the cart is located in the medicine rack operating area.

8. The medicine collecting system according to Claim 5,
wherein the medicine presenting unit is provided in the cart.

9. The medicine collecting system according to Claim 1,
wherein the cart further includes, for each of the housing units, a tray detecting unit configured to detect whether or not the tray in the housing unit is pulled out, and
the medicine presenting unit configured to present a type and a number of the second medicines to be supplied to the tray that is pulled out and detected by the tray detecting unit.

10. A cart used for a medicine collecting system that assists in collecting of a medicine comprising:
a plurality of housing units each configured to house a tray to which a first medicine is dispensed;
a light emitting unit provided at a position corresponding to each housing unit; and
a cart processing unit configured to light the light emitting unit corresponding to the housing unit housing the tray that requires supply of a second medicine on the basis of externally-received information on the second medicine.

11. The cart according to Claim 10, further comprising
a medicine presenting unit configured to present a type and a number of the second medicines to be supplied to the tray corresponding to the externally-received information.
